# EUROPÄISCHE PATENTANMELDUNG

(11) **EP 2 478 870 A1**
(43) Veröffentlichungstag der Anmeldung: **25.07.2012**
(21) Anmeldenummer: 12150656.2
(22) Anmeldetag: 10.01.2012
(51) Int. Cl.: A61F 2/24

(54) **Medizinisches Klappenimplantat, insbesondere Herzklappenimplantat, zur Implantation in einen tierischen und/oder menschlichen Körper**

(30) Priorität: 24.01.2011 US 201161435359 P
(71) Anmelder: Biotronik AG, 8180 Bülach (CH)
(72) Erfinder: Rzany, Alexander, 90449 Nürnberg (DE); Fringes, Matthias, 91522 Ansbach (DE); Borck, Alexander, 91086 Aurachtal (DE); Schmiedl, Robert, 96114 Hirschaid (DE); Harder, Claus, 91080 Uttenreuth (DE)
(74) Vertreter: Lindner-Vogt, Karin L.

(57) **Zusammenfassung**

Die Erfindung betrifft ein medizinisches Klappenimplantat (10a-d), insbesondere ein Herzklappenimplantat (12a-d), zur Implantation im tierischen und/oder menschlichen Körper mit einem Grundkörper (14a-d), der zumindest eine Wand (16a-d) aufweist.

Es wird vorgeschlagen, dass ein Bereich (18a-d) der zumindest einen Wand (16a-d) des Grundkörpers (14a-d) aushärtbar ist.

## Beschreibung

Die Erfindung betrifft ein medizinisches Klappenimplantat, insbesondere ein Herzklappenimplantat, zur Implantation in einen tierischen und/oder menschlichen Körper nach dem Oberbegriff des Patentanspruchs 1.

In der Medizin kommen Klappenimplantate zum Einsatz, die zur Erfüllung von Ersatzfunktionen permanent oder zumindest für einen längeren Zeitraum in einen tierischen und/oder menschlichen Körper eingebracht werden. Bekannt sind z. B. Herzklappenimplantate, wie etwa Aortenklappenimplantate, welche die Funktion der natürlichen Aortenklappe übernehmen. Hierbei wird das Klappenimplantat nach Expansion der Implantatstruktur sofort nach der Implantation fixiert und nimmt die Position der natürlichen Aortenklappe ein.

Ein häufiges Problem ist hierbei, dass das Implantat mit einer Fehlstellung fixiert wird, was zu einem Versagen des Implantats führen kann. Dies tritt beispielsweise oft bei Kalzifizierung, d.h. der Ablagerung von Kalziumsalzen, insbesondere Kalziumphosphat (Hydroxyapatit) an den Strukturen des Herzens und insbesondere bei einer stark asymmetrisch kalzifizierten Aortenstenose auf.

Der Erfindung liegt die Aufgabe zugrunde, ein medizinisches Klappenimplantat zu schaffen, das exakt und zuverlässig an einer Implantationsstelle implantiert werden kann. Die Aufgabe wird erfindungsgemäß durch die Merkmale des Patentanspruchs 1 gelöst. Günstige Ausgestaltungen und Vorteile der Erfindung ergeben sich aus den weiteren Ansprüchen, der Zeichnung und der Beschreibung.

Die Erfindung geht aus von einem medizinischen Klappenimplantat, insbesondere einem Herzklappenimplantat, zur Implantation im tierischen und/oder menschlichen Körper mit einem Grundkörper, der zumindest eine Wand aufweist.

Es wird vorgeschlagen, dass zumindest ein Bereich der zumindest einen Wand des Grundkörpers aushärtbar ist. Durch die erfindungsgemäße Ausgestaltung kann ein Klappenimplantat bereitgestellt werden, das optimal positioniert und schonend verankert werden kann. Ferner kann es vorteilhaft auf die Parameter bzw. an anatomischen Gegebenheiten einer Implantationsstelle, wie eine Kalzifizierung einer Blutgefäßwand und/oder eines Annulus, und/oder eine andere, angeborenen und/oder krankhafte Anomalie der Implantationsstelle angepasst werden. Des Weiteren erlaubt es eine optimale Platzierung an der Stelle der defekten physiologischen Klappe. Zudem wird eine Repositionierung und dauerhafte Fixierung *in vivo* ermöglicht. Ferner kann ein Druckgradient eines auf das medizinische Klappenimplantat wirkenden Fließmediums, wie beispielsweise Blut, homogen gehalten werden, was vorteilhaft zu einer geringen Materialbelastung des Klappenimplantats und dadurch zu einem geringen Ermüdungsrisiko durch gleichmässiges Öffnen der Klappe führt. Dies wiederum resultiert in einer langen Lebensdauer der Segel und damit der Klappe. Des Weiteren können durch die bessere Funktionalität der Klappe, die so einem höheren Druckgradienten standhalten kann, bei asymmetrisch kalzifiziertem Annulus bessere klinische Ergebnisse gegenüber den herkömmlichen Klappenimplantaten erzielt werden. Durch die erfindungsgemäße Ausgestaltung kann ferner eine Symmetrie in der Flussdynamik des Fließmediums erhöht werden, was das weitere Kalzifizierungsrisiko vorteihaft verringert. Ferner ist das Klappenimplantat strömungsmechanisch optimiert, wodurch turbulente Strömungen des Fließmediums reduziert werden können, was wiederum in einer geringeren Neigung zur Thrombenbildung resultiert.

In diesem Zusammenhang soll unter einem "Klappenimplantat" insbesondere ein Körper verstanden werden, der zumindest eine Ersatzfunktion eines Rückschlagventils, permanent oder für einen längeren Zeitraum ab Implantation, erfüllt. Denkbar wären hier sämtliche, dem Fachmann als zweckdienlich erscheinende medizinische Klappenimplantate, die in einen Hohlraum, wie beispielsweise einen Verdauungstrakt, einen Bronchialtrakt und/oder ein Blutgefäß, wie insbesondere eine Arterie und/oder eine Vene, des Körpers eingebracht werden. Unter einem "Herzklappenimplantat" soll hier insbesondere ein Implantat mit einer Implantatstruktur, wie beispielsweise eine Pulmonalklappe, eine Mitralklappe, eine Trikuspidalklappe und insbesondere eine Aortenklappe aus natürlichem und/oder künstlichem Material verstanden werden. Generell wäre jedoch jede andere, dem Fachmann für sinnvoll erscheinende Implantatstruktur denkbar.

Ferner soll in diesem Zusammenhang unter einem "Grundkörper" insbesondere eine Struktur, wie beispielsweise ein Zylinder bzw. ein Hohlzylinder, verstanden werden, die im Wesentlichen eine Gestalt und/oder eine Form des Klappenimplantats bildet. Hierbei soll der Schutzbereich nicht auf eine kreisrunde Kontur beschränkt sein, eckige oder ovale Formen sollen ebenso unter den Schutzbereich fallen. Zudem kann der Hohlzylinder auch Öffnungen in seiner Mantelfläche aufweisen. Der Grundkörper wird bevorzugt von einem schlauchförmigen Polymerzylinder gebildet. Dieser isoliert die defekten humanen Klappen vom neuen, zumeist tierischen, Klappenmaterial des Klappenimplantats, wodurch eine Kontaktfläche zwischen der künstlichen Klappe und einer Aortenwand gewebeschonend minimiert wird. Ferner kann dadurch effektiv eine Kalzifizierung des tierischen oder künstlichen Klappenmaterials verhindert werden, die bei direktem Kontakt von den defekten humanen Klappen auf die tierischen oder künstlichen Klappen überspringen könnte.

Ferner ist das Klappenimplantat bevorzugt passiv expandierbar bzw. mittels einer von außen zugeführten Kraft expandierbar bzw. plastisch verformbar. Konstruktiv einfach kann die passive Expansion mittels eines Ballonkatheters erfolgen, wobei der Grundkörper auf einen Ballonkatheter crimpbar ist.

Unter einer "Wand des Grundkörpers" soll insbesondere eine Außenwand des Grundkörpers verstanden werden und bevorzugt die Mantelfläche des Grundkörpers, die im implantierten Zustand in Richtung der Implantationsstelle bzw. von einem Mittelpunkt des Grundkörpers in radialer Richtung nach außen weist. Die Wand ist im implantierten Zustand für einen Kontakt mit der Implantationsstelle bzw. einem Annulus vorgesehen bzw. ist in Kontakt mit dem Gewebe der Implantationsstelle. Bevorzugt erstreckt sich der Bereich der Wand zumindest in Umfangsrichtung über den gesamten Umfang des Grundkörpers. Ferner erstreckt sich der Bereich der Wand bevorzugt entlang einer gesamten axialen Länge des Grundkörpers.

In diesem Zusammenhang soll unter "aushärtbar" insbesondere verstanden werden, dass ein Material aus dem der Bereich der Wand gefertigt ist, von einem ersten, weniger viskosen Zustand in einen zweiten, höher viskosen, bevorzugt festen Zustand übergeht. Der Zustandsübergang kann von jedem dem Fachmann als sinnvoll erscheinenden physikalischen, chemischen oder elektrischen Faktor, wie beispielsweise Zeit, Temperatur, Strahlung (IR-, VIS-, UV-, gamma-, radioaktive Strahlung), Ultraschall, Magnetismus, Strom, pH-Wert-, Konzentrations- und/oder Ladungsänderung abhängig sein. Das Material wird vorteilhafterweise stärker polymerisiert und/oder vernetzt.

Des Weiteren wird vorgeschlagen, dass der Bereich der zumindest einen Wand des Grundkörpers mittels UV-Strahlung aushärtbar ist. Bevorzugt ist hierbei eine von außen steuerbare UV-Lichtquelle in dem Ballonkatheter positioniert, die unmittelbar nach Dilatation ein schnelles Aushärten des Polymerschlauchs ermöglichen kann. Die UV-Strahlung liegt bevorzugt im Wellenlängenbereich von 240 - 400 nm, besonders bevorzugt im Bereich von 280 - 375 nm. Durch die Realisierung der UV-induzierten Aushärtung kann eine Aushärtzeit des Grundkörpers vorteilhaft gegenüber Klappenimplantaten des Stands der Technik verkürzt werden. Ferner kann dadurch realisiert werden, dass der unausgehärtete Zustand des Grundkörpers einen ausreichend langen Zeitraum erhalten bleibt, um eine gute Anpassung des Klappenimplantats an eine Herzmorphologie und/oder eine erforderliche Repositionierung zu gewährleisten.

Vorteilhafterweise ist der zumindest eine Bereich der zumindest einen Wand des Grundkörpers aus einem aushärtbaren Material ausgewählt aus einer Gruppe bestehend aus: Acrylaten, Methacrylaten, Cyanacrylaten, Epoxiden, Urethanen, Acrylamiden, Acylsäuren hergestellt. Bevorzugt ist der Grundkörper vollständig bzw. zu 100% von einem UVaushärtenden elastischen Polymerzylinder oder -schlauch gebildet. Es kommen vorteilhaft hoch flexible Acrylate und/oder Cyanacrylate sowie Methacrylate zum Einsatz, die in einem Zeitraum von 1 - 60 sec bei einer Zufuhr von 1 - 10 J/cm² aushärten. Grundsätzlich wäre auch jede dem Fachmann für zweckdienlich erscheinende Mischung der Materialien denkbar. Zudem können auch andere Materialien, wie beispielsweise Gießharze, Harze allgemein, Polyethylen (PET), Polykarbonat (PC), Polyamid (PI), PEBAX®, PVP, PA11, Silikonkautschuk oder PVC beigemischt sein. Ferner ist es auch denkbar andere Substanzen, wie Weichmacher (z. B. Triethanolamin), Kristallisationskeime (z. B. N-Vinylpyrrolidon), Farbstoffe (z. B. Eosin Y) Radikalstarter und/oder Röntgenkontrastmittel beizumischen. Durch die erfindungsgemäße Ausgestaltung kann das Aushärten konstruktiv einfach erfolgen. Ferner kann ein geringer Implantationsdurchmesser realisiert werden, da das Klappenimplantat keinen oder wenig Metallgehalt hat. Dadurch ergibt sich auch ein sehr flexibel verformbares Klappenimplantat. Des Weiteren findet kein direkter Kontakt von Metallen mit der Herzwand statt, wodurch es zu einer geringeren Interferenz mit der elektrischen Erregungsausbreitung am Herzen gegenüber Klappenimplantaten des Stands der Technik kommt. Zudem können durch eine Variation der Materialien verschiedene Grundkörper zur Verfügung gestellt werden, die je nach Anwendungsfall flexibel einsetzbar sind.

Ferner ist es vorteilhaft, wenn der Grundkörper dazu vorgesehen ist, einen Unterschied in einer Form eines Innenquerschnitts des Grundkörpers und einer Querschnittsfläche einer Implantationsstelle auszugleichen. Unter "vorgesehen" soll insbesondere speziell ausgestattet, ausgelegt und/oder vorbereitet verstanden werden. In diesem Zusammenhang soll unter einer "Form des Innenquerschnitts des Grundkörpers" insbesondere eine weitestgehend runde bzw. zylinderförmige Form verstanden werden, sodass sich Segel der Klappe komplikationslos Öffnen und Schließen können. Unter einer "Querschnittsfläche einer Implantationsstelle" soll hier insbesondere eine stark asymmetrisch bzw. unrunde Stelle insbesondere mit beispielsweise einer kalzifizierten Aortenstenose verstanden werden. Der Grundkörper passt somit die mögliche uneinheitliche Form eines Außendurchmessers des Grundkörpers vorteilhaft an die Querschnittsfläche der Implantationsstelle an, wodurch das Implantat in besonderem Maße die örtlichen Gegebenheiten an der Implantationsstelle berücksichtigt. Dadurch kann vorteilhaft eine Unsymmetrie der Blutgefäßwand bzw. des Annulus ausgeglichen werden und dennoch eine weitestgehend runde, symmetrische Innenform des Grundkörpers zur benötigten fehlerfreien und komplikationslosen Funktion von Segeln der Klappe beibehalten werden.

In einer weiteren Ausgestaltung der Erfindung wird vorgesehen, dass der Grundkörper zumindest ein Mittel zur mechanischen Verstärkung aufweist. Diese Mittel bzw. das Stützmittel kann eine metallische (z. B. Nitinol) oder polymere Struktur sein, die in das aushärtbare Polymer integriert ist und/oder eine Ausgestaltung, wie beispielsweise eine Verdickung, ein Wulst und/oder eine Ringstruktur, sein, die an den Grundkörper angeformt und/oder in diesem ausgeformt ist. Grundsätzlich kann das Mittel zu mechanischen Verstärkung auch mittels einer anderen Art, wie einem Kraft-, Stoff-, oder Formschluss, mit dem Grundkörper verbunden sein. Das Mittel zur mechanischen Verstärkung dient insbesondere einer Unterstützung einer Fixierung des Klappenimplantats, wodurch vorteilhaft auf beispielsweise dornartige Verankerungsmechanismen gemäß des Stands der Technik, die das Gewebe reizen und Entzündungen hervorrufen können, verzichtet werden kann. Ferner kann das Mittel zur mechanischen Verstärkung als Positioniermittel zu einer exakten Positionierung des Klappenimplantats verwendet werden.

Ferner wird vorgeschlagen, dass der Grundkörper einen Stent umfasst. Hierbei bildet der Grundkörper bevorzugt den Stent, der somit aus einem ballonexpandierbaren aushärtbaren Polymer besteht. Durch die Ausführung des Klappenimplantats als Stent bzw. dadurch dass der Grundkörper einen Stent umfasst, kann eine konstruktiv einfach zu implantierende Struktur bereitgestellt werden. Durch diese Ausgestaltung kann ein Klappenimplantat mit einer kurzen Bauhöhe ermöglicht werden, wodurch gegenüber den Implantaten des Stands der Technik ein geringeres Risiko besteht z. B. den Einstrom des Bluts in die ostialen Abgänge der Koronararterien zu behindern oder sie sogar ganz zu verschließen.

Ferner wäre es vorteilhaft wenn das Klappenimplantat zusätzlich zu dem Grundkörper einen weiteren Stent umfasst. Dieser Stent ist relativ zu dem Grundkörper radial innen angeordnet und kann von jedem dem Fachmann für einsetzbar erachteten Stent, beispielsweise einem ballondilatierbaren oder selbstexpandierenden Stent aus einem elastischen oder superelastischen Material, etwa einem metallischen Material (Eisen, Magnesium, Nickel, Wolfram, Titan, Zirkonium, Niob, Tantal, Zink, Silizium, Lithium, Natrium, Kalium, Kalzium, Mangan), aus einer Kombination von mehreren metallischen Materialien, aus einem Formgedächtnis-Material, wie Nitinol, aus medizinischem Edelstahl, wie Co-Cr-Stahl, aus Kunststoff, aus einer Keramik und/oder aus einem biodegradierbaren Material gefertigt sein.

Des Weiteren kann es vorteilhaft sein, wenn das Mittel zur mechanischen Verstärkung von zumindest einer Ringstruktur gebildet ist, wodurch das Klappenimplantat durch eine Selbstpositionierung physiologisch optimal am Annulus platziert und fixiert werden kann. Die Ringstruktur kann komplett aus demselben aushärtbaren Material wie der Grundkörper und/oder sie kann von einem hohlen Ring gebildet sein. Im letzten Fall ist eine Umhüllung des Rings aus einem flexiblen Polymermaterial gefertigt, wie bevorzugt Polyethylen (PET), Polykarbonat (PC), Polyimid (PI), PEBAX®, PVP, PA11, PVC und/oder jedes andere, dem Fachmann für einsetzbar erscheinendes Material.

Eine genaue und sichere Fixierung kann vorteilhaft erreicht werden, wenn die Ringstruktur an einem proximalen Ende und/oder einem distalen Ende des Grundkörpers angeordnet ist. In diesem Zusammenhang soll unter einem "proximalen Ende" insbesondere ein Ende des Grundkörpers verstanden werden, das im Implantationsprozess zum Bediener weist. Demnach ist ein "distales Ende" ein Ende das vom Bediener weg weist. Bevorzugt ist je eine Ringstruktur an dem proximalen Ende und an einem distalen Ende angeordnet, wodurch die Gestalt des Grundkörpers besonders zuverlässig an die Form der Implantationsstelle bzw. dem Annulus angepasst werden kann.

Zudem wird vorgeschlagen, dass die Ringstruktur mit einem aushärtbaren Material befüllbar ist. Herbei kann das aushärtbare Material im Befüllungszustand flüssig sein. Auch hier kann der Zustandsübergang von beispielsweise flüssig und/oder weniger viskos zu viskos bzw. fest von jedem dem Fachmann als sinnvoll erscheinenden physikalischen, chemischen oder elektrischen Faktor abhängig sein, jedoch bevorzugt von Zeit und/oder UV-Strahlung. Es kommt vorteilhaft eine vernetzbare Polymerlösung zum Einsatz. Bevorzugt kann sich die Polymerlösung dabei aus mehreren Komponenten zusammensetzen, deren relatives Verhältnis und Zusammensetzung eine gezielte Einstellung physikalischer Eigenschaften vorteilhaft erlaubt. Optional können hierbei auch unterschiedliche Eigenschaften für die einzelnen Ringstrukturen realisiert werden. Vorteilhaft lässt sich über das Verhältnis von polymeren zu monomeren Anteilen sowie die Konzentration von chemisch aktivierenden Substanzen (Initiatoren für die Polymerisation) unmittelbar die Reaktionsgeschwindigkeit beeinflussen.

Andere Eigenschaften lassen sich durch den Zusatz von nicht an der Polymerisation beteiligten Füllstoffen erreichen, wie beispielsweise Röntgensichtbarkeit durch Zusatz von Röntgenkontrastmittel (z. B. Bariumsulfat), Erhöhung der Flexibilität der auspolymerisierten Füllsubstanz durch Zusatz von elastischen Polymeren (z. B. Gummipartikel), Erhöhung der mechanischen Festigkeit der auspolymerisierten Füllsubstanz durch Zusatz von Füllstoffen wie in Knochenzementen eingesetzter Hydroxylapatit und/oder andere dem Fachmann für vorteilhaft erachtete Eigenschaften. Optional ist zur Positionierung auch ein temporäres Befüllen der Ringstrukturen mit physiologischer Kochsalzlösung möglich, welche erst in einem zweiten Schritt durch die aushärtbare Polymerlösung ersetzt wird. Zum Befüllen der Ringstrukturen kann ein Kathetersystem wie in WO 2006/105190 A2 beschrieben zum Einsatz kommen, welches sich durch eine lösbare Verbindung auszeichnet. Alternativ können von außen, über das Kathetersystem kontrollierbare Ventile, zum Einsatz kommen, welche eine lösbare Verbindung zwischen Ringstrukturen und Kathetersystem herstellen.

Vorteilhafterweise weist das medizinische Klappenimplantat zumindest zwei Ringstrukturen auf, die unabhängig voneinander befüllbar sind, wodurch die Positionierung des Klappenimplantats besonders präzise erfolgen kann. Ferner können so verschiedene Eigenschaften der Ringstrukturen bzw. deren Füllsubstanz einfach realisiert werden. Es wird bspw. zuerst die Ringstruktur am distalen Ende des Grundkörpers über das mit der implantierbaren Klappe verbundene Kathetersystem befüllt. Durch ein gezielt langsames Inflatieren lässt sich dieser nach und nach optimal positionieren und bildet so einen Anschlag für die optimale Platzierung der künstlichen minimalinvasiv implantierbaren Herzklappe. Erst zeitlich nach der korrekten Platzierung der distalen Ringstruktur wird die Ringstruktur am proximalen Ende des Grundkörpers befüllt und die Herzklappe eigenständig im Bereich der durch die defekte physiologische Herzklappe gebildete Engstelle zentriert. Die Lösung zum Befüllen kann über das Kathetersystem teilweise zurückgezogen werden um den Druck zu erniedrigen, wodurch vorteilhaft eine Repositionierung der Ringstruktur(en) z. B. bei Fehlpositionierungen ermöglich wird. Nach Verifikation der korrekten Positionierung wird das Klappenimplantat durch Vernetzung der zum Befüllen verwendeten Lösung dauerhaft fixiert. Hierdurch kann ferner konstruktiv einfach ein Versagen des Implantats verhindert werden.

Des Weiteren weist das medizinische Klappenimplantat vorteilhaft eine Klappe auf, die in einer axialen Richtung über der Ringstruktur am proximalen Ende des Grundkörpers angeordnet ist. Durch diese Anordnung wird ein Klappenimplantat bereitgestellt, das während der Implantation eine vorteilhafte Selbstfindung/Selbstpositionierung der Klappe aufweist. Grundsätzlich könnte die Klappe auch axial zwischen zwei Ringstrukturen angeordnet sein. Dies resultiert in einer besonders zuverlässigen Justierung der Klappe. Bei Positionierung zwischen den Ringstrukturen kann die Klappe auch an dem Grundkörper angebracht sein und/oder mit einer Ringstruktur oder beiden Ringstrukturen verbunden sein. Hierbei kann die Klappe mittels jeder dem Fachmann für sinnvoll erachtete Verbindungsart, wie beispielsweise Nähen und/oder Kleben mit der Ringstruktur oder dem Grundkörper verbunden sein.

Eine bevorzugte Weiterbildung besteht darin, dass zumindest zwei Ringstrukturen im bestimmungsgemäßen Endzustand in Fließrichtung eines Fließmediums axial vor und hinter dem Annulus angeordnet sind. Unter einer "Fließrichtung eines Fließmediums" soll hier insbesondere die wissenschaftlich bekannte Fließrichtung von arteriellem und/oder venösem Blut im Herzen und besonders vorteilhaft im Falle der Aortenklappe der Fluss von Blut vom linken Ventrikel in die Aorta verstanden werden. Hierbei handelt es sich bei dem Annulus bevorzugt um den Aortenannulus. Durch die Realisierung der erfindungsgemäßen Anordnung kann das Klappenimplantat besonders gut an die Anatomie des Herzens bzw. einer Herzklappenregion mit beispielsweise einem Aortenbulbus angepasst werden.

Es wird zudem vorgeschlagen, dass zwischen zwei Ringstrukturen zumindest eine Kommunikationsstruktur angeordnet ist. In diesem Zusammenhang soll unter einer "Kommunikationsstruktur" insbesondere eine Struktur mit einem oder mehreren hohlförmigen Elementen, wie ein Kanalsystem und/oder ein Hohlraum verstanden werden, mittels dem die beiden Ringstrukturen in Kontakt miteinander stehen. Hierbei kann das Kommunikationssystem unabhängig vom Grundkörper bzw. dem Zylinder ausgeführt sein oder in diesen integriert sein. Bei Letzterem ist der Zylinder beispielsweise doppelwandig mit einem Hohlraum dazwischen ausgeführt. Hierbei ist der Füllstoff für die Ringstrukturen und die Kommunikationsstruktur baugleich. Die Verbindungen zwischen den einzelnen Funktionseinheiten des Klappenimplantats erlauben konstruktiv einfach den Austausch der vernetzbaren Polymerlösung und damit eine vorteilhaft eigenständige Anpassung des Klappenimplantats an die Herzgeometrie. Es wird deshalb als wesentlicher Vorteil eine weitgehend eigenständige Positionierung der Klappe im Annulus während der Befüllung mit der aushärtbaren Polymerlösung erreicht.

Eine weitere Ausgestaltung der Erfindung sieht vor, dass zumindest an einer Ringstruktur an dem proximalen Ende des Grundkörpers und/oder an einem proximalen Ende einer Klappe eine Stützstruktur angeordnet ist. Diese Stützstruktur dient bevorzugt der Stützung, Positionierung und der proximalen mechanischen Fixierung der Klappe. Sie ist beispielsweise ausgeführt als Metallgerüst in der Form eines Drahtgeflechts bevorzugt aus Nitinol und/oder als eine weitere bzw. dritte Ringstruktur, die unabhängig von den anderen Funktionseinheiten des Klappenimplantats positionierbar, befüllbar und fixierbar ist. Durch die Stutzstruktur kann die relative Positionierung der Ringstrukturen und der Klappensegel sichergestellt werden, um ein exaktes und komplikationsloses Öffnungs- und/oder Schließverhalten der Segel zu gewährleisten. Die Ausführung als weitere Ringstruktur ist insbesondere dann von Vorteil, wenn die diese Ringstruktur eine hohe mechanische Flexibilität aufweist und dadurch eine gute Adaption an die physiologischen Gegebenheiten des Herzen erlaubt. Dadurch können optimale Strömungseigenschaften und eine geringe mechanische Einschränkung der Herzbewegung erreicht werden.

Besonders vorteilhaft ist eine Ausgestaltung des medizinischen Klappenimplantats als Aortenklappe, wodurch eine ausgefeilte Ersatzstruktur für die am häufigsten mit Fehlfunktionen behaftete Herzklappe bereitgestellt werden kann. Auch können Komplikationen wie z. B. Störungen der Mitralklappe oder die Notwendigkeit eines Herzschrittmachers günstigerweise reduziert werden. Ebenso ist eine Ausgestaltung als Pulmonalklappe, Trikuspidalklappe oder auch eine Ausgestaltung als Mitralklappe denkbar.

Vorteilhaft kann am Implantat eine ablagerungshemmende, insbesondere kalzifizierungshemmende, Beschichtung vorgesehen sein, insbesondere Homocysteinsäure. Damit kann die Gefahr eine Störung oder ein Funktionsausfall des Klappenimplantats weiter verringert werden.

Die Erfindung ist nachfolgend beispielhaft, anhand von einem in Zeichnungen dargestelltes Ausführungsbeispiel näher erläutert. Es zeigen:
- Fig. 1: ein erfindungsgemäßes Klappenimplantat im expandierten Zustand,
- Fig. 2: das Klappenimplantat der Fig. 1 montiert auf eine Implantationsvorrichtung,
- Fig. 3: das Klappenimplantat der Fig. 1 bei einer Implantation an einer Implantationsstelle,
- Fig. 4: das Klappenimplantat der Fig. 1 im implantierten Zustand an einem Annulus,
- Fig. 5: eine schematische Darstellung eines Schnitts entlang der Linie V-V der Fig. 4 durch die Aortenwand mit Aufsicht auf die Segel des Klappenimplantats,
- Fig. 6: eine schematische Darstellung des Einführens der Implantatvorrichtung mit dem Klappenimplantat der Fig. 1 an einer Implantationsstelle,
- Fig. 7: ein alternatives Klappenimplantat im implantierten Zustand an einem Annulus,
- Fig. 8: ein weiteres alternatives Klappenimplantat im implantierten Zustand an einem Annulus in einer schematischen Darstellung,
- Fig. 9: eine schematische Darstellung einer mechanischen Stützstruktur für die Klappensegel mit Fixiermöglichkeit auf einer proximalen Ringstruktur,
- Fig. 10: ein drittes alternatives Klappenimplantat im implantierten Zustand an einem Annulus in einer schematischen Darstellung,
- Fig. 11: Schematischer Ablauf der Vernetzung ungesättigten Polyester,
- Fig. 12: Initiator-Beschleuniger-Kombinationen bei UP-Harzsystemen und
- Fig. 13: Schema der Peroxidspaltung zum Polymerisationsstart.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

Zur Vermeidung unnötiger Wiederholungen wird bei nicht näher beschriebenen Elementen in einer Figur auf die jeweilige Beschreibung der Elemente in vorstehenden Figuren verwiesen.

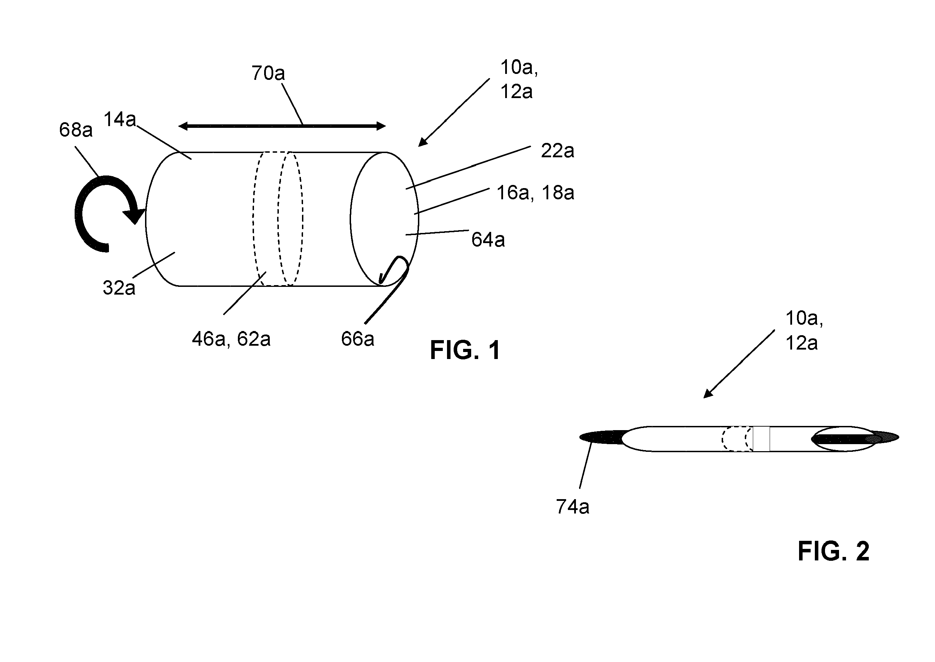

Fig. 1 zeigt ein medizinisches Klappenimplantat 10a bzw. ein Herzklappenimplantat 12a, zur Implantation im tierischen und/oder menschlichen Körper im expandierten Zustand. Das Klappenimplantat 10a weist einen Grundkörper 14a mit einer Wand 16a auf, wobei der Grundkörper 14a von einem Hohlzylinder 64a in der Form eines Polymerschlauchs und die Wand 16a vom kompletten Zylindermantel gebildet ist. Grundsätzlich wäre es auch denkbar, dass die Wand 16a lediglich von einer Außenwand des Hohlzylinders 64a gebildet wird. Der Grundkörper 14a umfasst einen Stent 32a bzw. bildet einen Stent 32a, wobei dessen Stentrahmen vollständig von dem elastischen Polymerschlauch gebildet wird. An einer inneren Mantelfläche 66a des Grundkörpers 14a ist eine Klappe 46a, ausgestaltet als Aortenklappe 62a, untrennbar befestigt. Die Wand 16a des Grundkörpers 14a weist einen Bereich 18a auf, der sich in einer Umfangsrichtung 68a über den gesamten Umfang des Grundkörpers 14a und entlang einer gesamten axialen Länge 70a erstreckt.

Der Bereich 18a und die Wand 16a sind hier identisch. Der Bereich 18a bzw. die Wand 16a ist aus einem aushärtbaren Material 22a hergestellt, das mittels UV-Strahlung 20a aushärtbar ist. Das Material 22a setzt sich zusammen aus einer Mischung von hoch flexiblen Acrylaten und Cyanacrylaten, der Triethanolamin, N-Vinylpyrrolidon und Eosin Y beigemischt ist. Hierbei werden beispielsweise einer Menge von 10 ml Acrylaten und Cyanacrylaten, die in jedem Mischungsverhältnis einsetzbar sind, 0,5 ml Triethanolamin und 50 µl N-Vinylpyrrolidon beigemischt in denen 0,3% Eosin Y gelöst ist. Durch eine solche Zusammensetzung kann die Vernetzungsreaktion auch ohne Temperaturerhöhung mit Hilfe einer UV-Lichtquelle 72a gestartet werden.

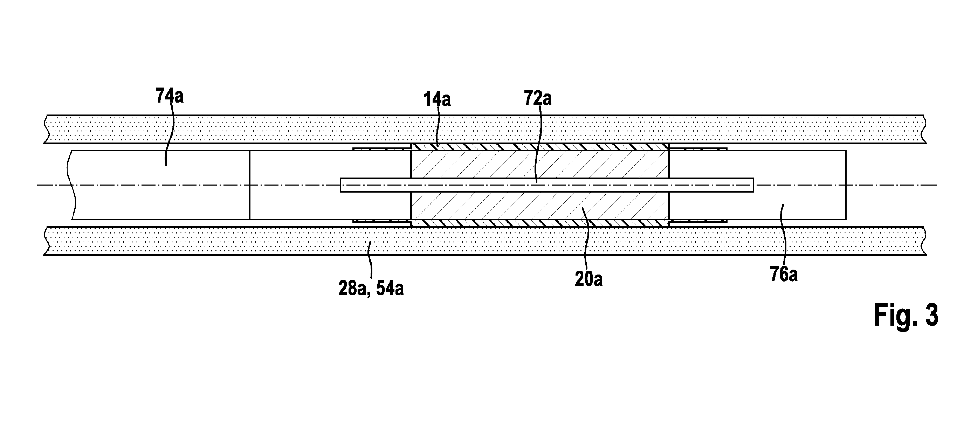

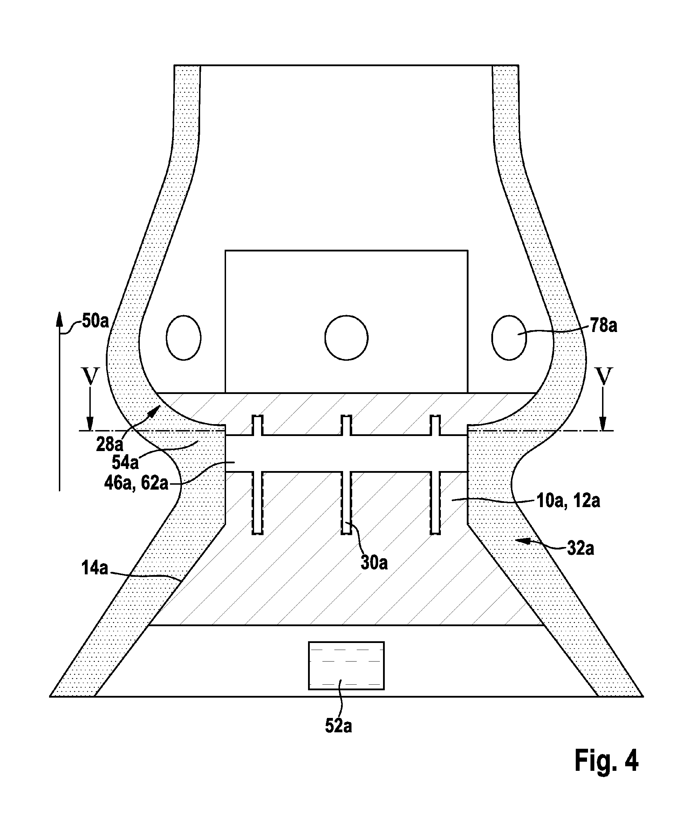

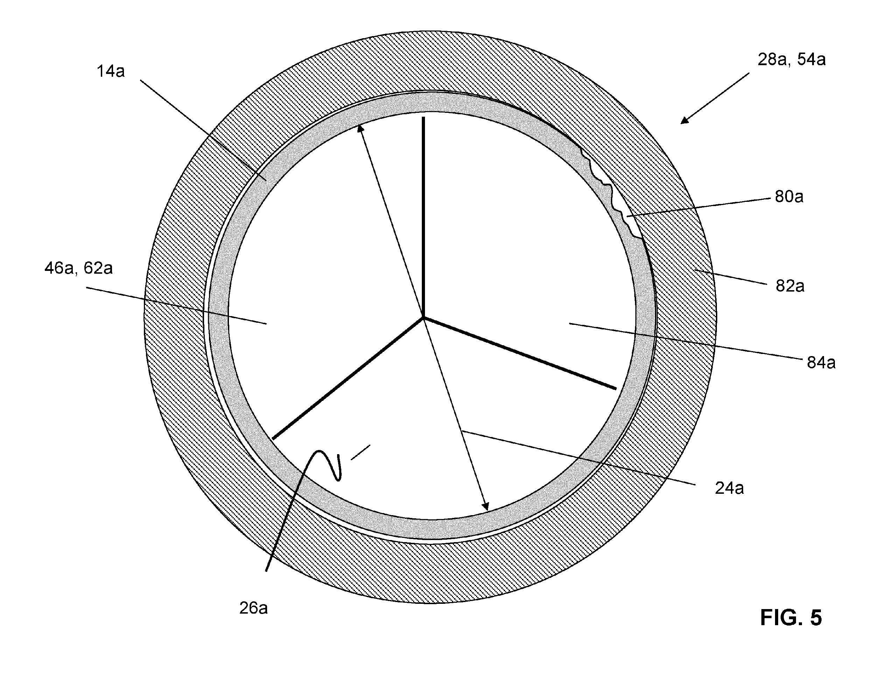

Wie in Fig. 2 zu sehen ist, ist das Klappenimplantat 10a für eine Implantation in seinem kollabierten Zustand auf eine Implantationsvorrichtung bzw. auf einen Ballonkatheter 74a gecrimpt. Fig. 3 zeigt in einer schematischen Darstellung die Implantationsvorrichtung und das Klappenimplantat 10a während der Implantation des Klappenimplantats 10a an einer Implantationsstelle 28a, wie einem Annulus 54a bzw. dem Aortenannulus des Herzens (siehe Fig. 4). Der Übersichtlichkeit halber ist die Klappe 46a hier nicht dargestellt. Bei der Implantation wird der Grundkörper 14a bzw. der Polymerschlauch mit einem Ballon 76a in der Implantationsstelle 28a dilatiert und dabei optimal an eine Gefäßanatomie angepasst. In dem Ballon 76a ist eine von außen steuerbare UV-Lichtquelle 72a positioniert, mit der über den Ballon 76a UV-Strahlung 20a mit einer Energie von 1 - 10 J/cm², eingestrahlt wird. Dadurch wird der Grundkörper 14a in einer Zeit zwischen 1 - 60 Sekunden ausgehärtet, sodass dieser nun seine Stützfunktion übernehmen kann. Nach der Aushärtung ist das Klappenimplantat 10a - trotz seiner niedrigen Bauhöhe - optimal fixiert und formschlüssig mit dem Annulus 54a verbunden, was strömungsmechanisch vorteilhaft ist. Dies ist in Fig. 4, die das Klappenimplantat 10a implantiert an dem Annulus 54a zeigt, zu sehen. Der Grundkörper 14a lässt durch seine kurze Bauhöhe die Abgänge 78a der Koronararterien frei und deckt die physiologische Klappe (nicht gezeigt) vollständig ab, was die künstliche Klappe 46a vor einem Überspringen einer Kalzifizierung 80a an der physiologische Klappe schützt (siehe Fig. 5).

Optional, wie dies in Fig. 4 gestrichelt angedeutet ist, kann der Grundkörper 14a ein Mittel 30a zur mechanischen Verstärkung in der Form einer metallischen Struktur aus Nitinol aufweisen. Das Mittel 30a ist in das aushärtbare Polymer integriert. Der Grundkörper 14a bzw. der Polymerschlauch ragt proximal und distal über das Mittel 30a hinaus. Grundsätzlich können mehrere Mittel 30a integriert sein, die auch als ein Gerüst verbunden sein können. Vorteilhaft ist dabei u.a., dass keine dornartigen Verankerungsmechanismen, wie diese bei Implantaten des Stands der Technik oft zum Einsatz kommen, notwendig sind, die das Gewebe reizen und Entzündungen hervorrufen können.

Wie aus der Fig. 5, die einen Schnitt entlang der Linie V-V der Fig. 4 durch eine Aortenwand 82a mit Aufsicht auf Segel 84a der Klappe 46a zeigt, hervorgeht, ist der Grundkörper 14a dazu vorgesehen, einen Unterschied in einer Form bzw. einer runde Form eines Innenquerschnitts 24a des Grundkörpers 14a und einer beispielsweise unregelmäßigen Querschnittsfläche 26a der Implantationsstelle 28a auszugleichen. Im Bereich einer Kalzifizierung 80a an der Aortenwand 82a kann sich der Grundkörper 14a während der Implantation durch seine flexible und verformbare Wand 16a an die Kontur der Kalzifizierung 80a anpassen ohne dass eine runde Geometrie der Klappe 46a beeinflusst wird. Dadurch können sich die Segel 84a unbehindert Öffnen und Schließen.

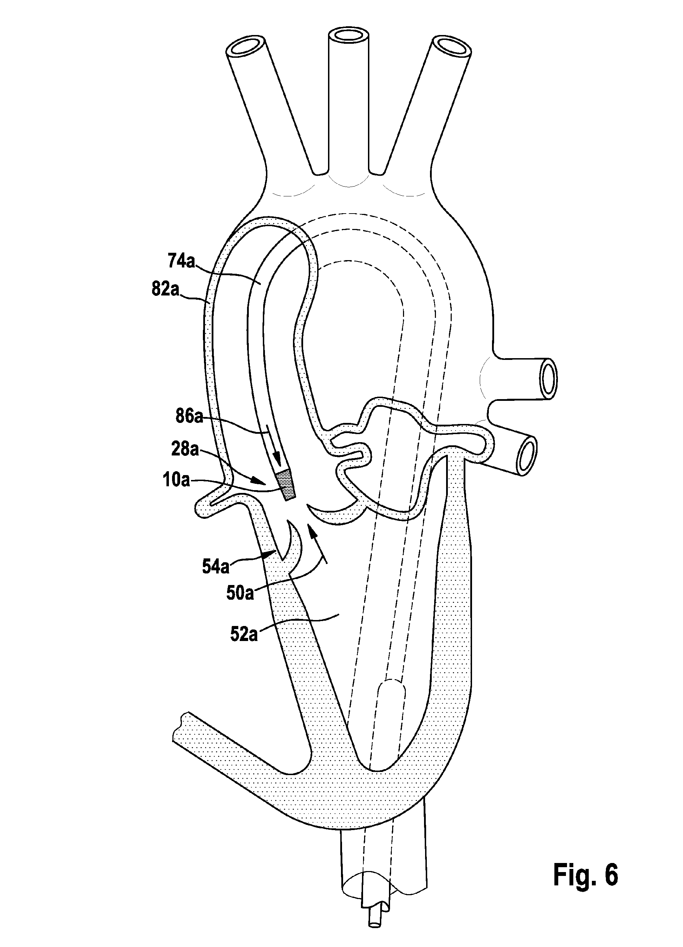

In der Fig. 6 ist das Einführen des medizinischen Klappenimplantats 10a mittels eines transfemoralen Zugangs schematisch in einer Teilschnittdarstellung illustriert. Die Implantationsvorrichtung bzw. der Ballonkatheter 74a mit dem Klappenimplantat 10a werden in an sich bekannter Weise an die Implantationsstelle 28a, z. B. den Annulus 54a der natürlichen Aortenklappe mit Segeln zugeführt. Hierbei ist eine Implantationsrichtung 86a entgegengesetzt zu einer Fließrichtung 50a eines Fließmediums 52a, wie Blut (siehe Fig. 4). Ist die korrekte Position erreicht, wird das Klappenimplantat 10a, wie in Fig. 3 gezeigt, mit dem Ballon 76a dilatiert, bis es seine exakte Position einnimmt (siehe Fig. 4). Ist dies geschehen wird das aushärtbare Material 22a bzw. das Polymer mittels UV-Strahlung 20a ausgehärtet und das Klappenimplantat 10a ist in seinem bestimmungsgemäßen Endzustand am Annulus 54a fixiert.

Es wird in der klinischen Praxis auch der so genannte transapikale Zugang verwendet, wobei die Klappe durch den Ventrikel hindurch implantiert wird. Hierbei würden sich bei den folgenden Beschreibungen jeweils die Begriffe "proximal" und "distal" vertauschen.

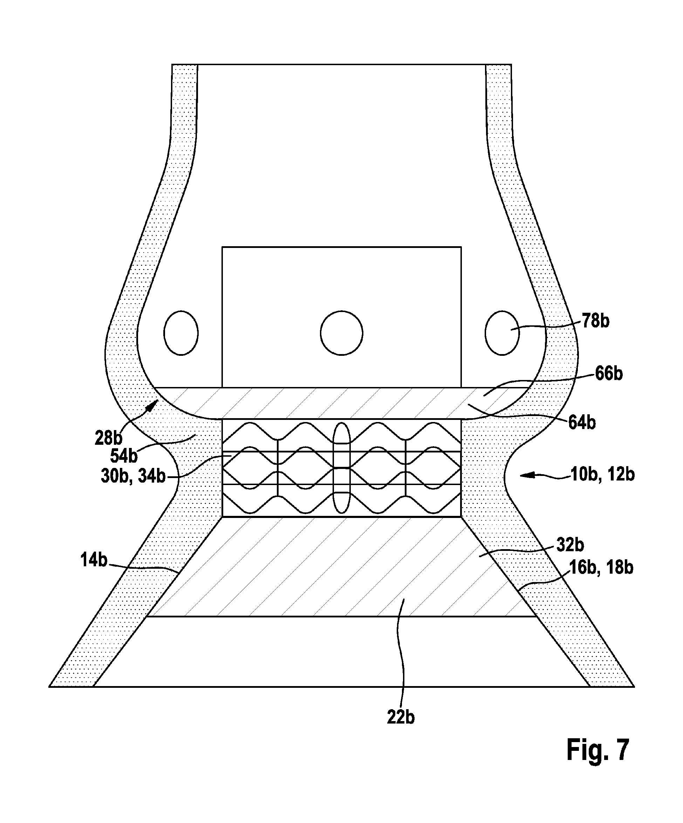

In den Figuren 7 bis 10 sind alternative Ausführungsbeispiele des medizinischen Klappenimplantats 10a dargestellt. Im Wesentlichen sind gleich bleibende Bauteile, Merkmale und Funktionen grundsätzlich mit den gleichen Bezugszeichen beziffert. Zur Unterscheidung der Ausführungsbeispiele sind jedoch den Bezugszeichen der Ausführungsbeispiele die Buchstaben a bis d hinzugefügt. Die nachfolgende Beschreibung beschränkt sich im Wesentlichen auf die Unterschiede zu dem Ausführungsbeispiel in den Figuren 1 bis 6, wobei bezüglich gleich bleibender Bauteile, Merkmale und Funktionen auf die Beschreibung des Ausführungsbeispiels in den Figuren 1 bis 6 verwiesen werden kann.

Fig. 7 zeigt in einer Ansicht schematisch ein alternatives medizinisches Klappenimplantat 10b bzw. ein Herzklappenimplantat 12b zur Implantation im tierischen und/oder menschlichen Körper mit einer nicht dargestellten künstlichen Klappe im implantierten Zustand in einer Implantationsstelle 28b bzw. einem Annulus 54b einer natürlichen Aortenklappe. Das Klappenimplantat 10b weist einen Grundkörper 14b mit einer Wand 16b auf, deren Bereich 18b, der übereinstimmt mit der Wand 16b und dem kompletten Grundkörper 14b, ein Material 22b aufweist, das mittels UV-Strahlung aushärtbar ist.

Zusätzlich zu dem Stent 32b, der von dem Grundkörper 14b selbst gebildet wird, umfasst das Klappenimplantat 10b bzw. der Grundkörper 14b einen weiteren Stent 34b als ein Mittel 30b zur mechanischen Verstärkung auf. Dieser Stent 34b ist an einer inneren Mantelfläche 66b des Hohlzylinders 64b des Grundkörpers 14b angeordnet und ist ebenso ballondilatierbar. Grundsätzlich könnte der Stent 34b auch selbstexpandierend ausgeführt sein. Zudem ist die künstliche Klappe an dem Stent 32b befestigt. Der Grundkörper 14b bzw. der Polymerschlauch ragt proximal und distal über den Stent 34b, aber nicht bis zu den Abgängen 78b der Koronararterien, hinaus, wodurch der Polymerschlauch bei der Dilatation proximal und distal des Stents 34b an die individuelle Morphologie angepasst wird. Nach exakter Positionierung wird er mittels einer UV-Lichtquelle ausgehärtet.

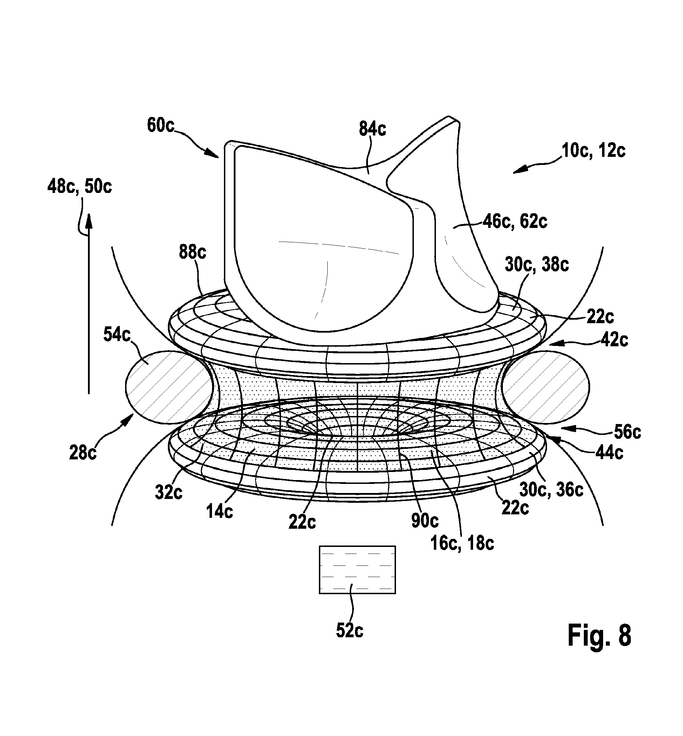

In der Fig. 8 ist ein weiteres alternatives Klappenimplantat 10c bzw. ein Herzklappenimplantat 12c zur Implantation im tierischen und/oder menschlichen Körper mit einer künstlichen Klappe 46c in der Form einer Aortenklappe 62c und mit einem Grundkörper 14c gezeigt. Der Grundkörper 14c, der von einem elastischen Polymerschlauch gebildet ist, weist eine Wand 16c auf, deren mit der Wand 16c und dem kompletten Grundkörper 14c übereinstimmender Bereich 18c aus einem mittels UV-Strahlung aushärtbaren Material 22c gefertigt ist. Der Grundkörper 14c, der einen Stent 32c bildet, weist an einem proximalen Ende 42c und an einem distalen Ende 44c je ein Mittel 30c zur mechanischen Verstärkung auf, wobei jedes Mittel 30c von einer Ringstruktur 36c, 38c gebildet ist. Im bestimmungsgemäßen Endzustand d.h. im implantierten Zustand an einer Implantationsstelle 28c sind die beiden Ringstrukturen 36c, 38c in Fließrichtung 50c eines Fließmediums 52c axial vor und hinter einem Annulus 54c angeordnet. Ferner ist die Ringstruktur 36c, 38c jeweils von einem hohlen Ring gebildet, dessen Umhüllung 88c aus einem flexiblen Polymer, wie Polyethylen, besteht.

Die Ringstrukturen 36c, 38c sind mit einem aushärtbaren Material 22c befüllbar (Materialien siehe unten) und unabhängig voneinander befüllbar. Es wird zuerst die Ringstruktur 36c am distalen Ende 44c über das mit dem Klappenimplantat 10c verbundene Kathetersystem befüllt. Durch gezielt langsames Inflatieren lässt sich dieser nach und nach optimal Positionieren. Erst wenn die distale Ringstruktur 36c korrekt sitzt, wird die Ringstruktur 38c am proximalen Ende 42c analog positioniert und befüllt. Bei beiden Vorgängen kann - um bei Bedarf den Druck zu erniedrigen - die Lösung zum Befüllen über das Kathetersystem teilweise zurückgezogen werden. Dies ermöglicht eine einfache Repositionierung des Implantats bei eventuellen Fehlpositionierungen, z. B. Undichtigkeiten. Die Ringstrukturen 36c, 38c stellen somit Positioniermittel dar. Generell können die Ringstrukturen 36c, 38c mit demselben Material 22c oder mit unterschiedlichen Materialien 22c befüllt werden.

Zwischen den zwei Ringstrukturen 36c, 38c ist eine Kommunikationsstruktur 56c in der Form eines Kanalsystems mit hohlen Elementen 90c angeordnet, worüber sich das Material 22c zwischen den Ringen verteilen kann. Die hohlen Elemente 90c können hierbei im Grundkörper 14c integriert sein oder auch von einer separaten Struktur gebildet sein (nicht gezeigt). Alternativ zu dem Kanalsystem kann der Grundkörper 14c auch doppelwandig ausgeführt sein, sodass zwischen den Wänden ein Hohlraum gebildet wird über den die Ringstrukturen 36c, 38c kommunizieren können. Für den Stoffaustausch müssen hierbei Öffnungen in die Umhüllungen 88c der Ringe eingebracht sein (nicht gezeigt). Alternativ können die Ringstrukturen 36c, 38c auch in eine hohle befüllbare Polymerfolie als Ausbuchtungen an den Enden 42c, 44c integriert sein. Ferner kann auf die Kommunikationsstruktur 56c auch ganz verzichtet werden.

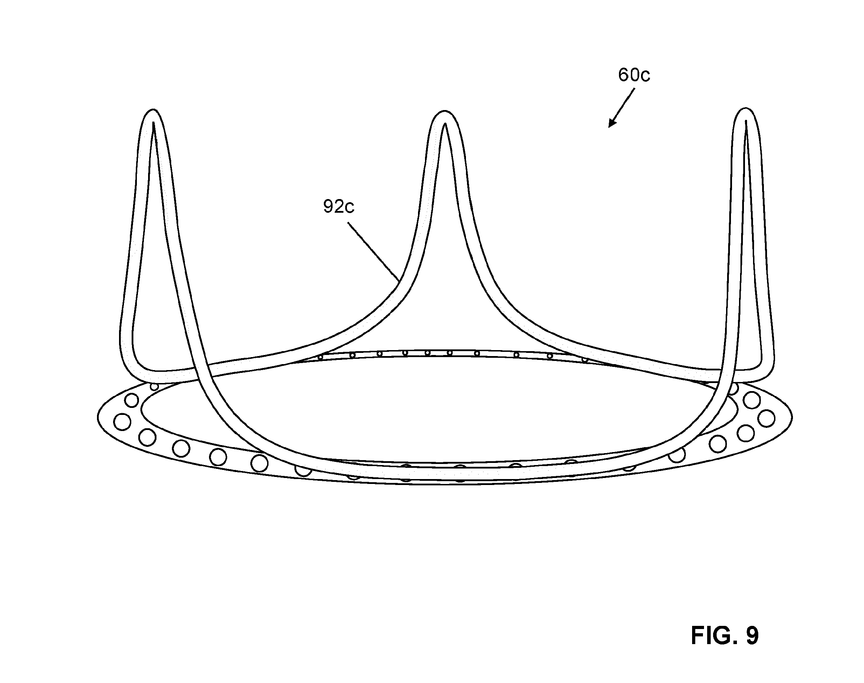

Die Klappe 46c ist in einer axialen Richtung 48c über der Ringstruktur 38c am proximalen Ende 42c des Grundkörpers 14c in der Aorta über dem Annulus 54c angeordnet. Zu einer oberen Fixierung von Segeln 84c der Klappe 46c ist an der proximalen Ringstruktur 38c eine mechanische Stützstruktur 60c in der Form eines Metallgerüsts 92c aus z. B. Nitinol befestigt (siehe Fig. 9). Diese stellt die relative Positionierung der Ringstruktur 38c und der Segel 84c sicher und gewährleistet das zuverlässige Öffnungs- und Schließverhalten der Segel 84c.

Das aushärtbare Material 22c zum Befüllen der Ringstrukturen 36c, 38c kann entweder mittels UV-Strahlung aushärtbar sein oder es kann nach einer gewissen Zeit selbst aushärten. Hierfür sind mehrere alternative Polymerlösungen denkbar:

### Beispiel 1 (UV-aushärtbare Polymerlösung)

Granuliertes Polymethacrylat (PMMA; 1 - 10 µm) wird mit Methylmethacrylat (bestehend aus 97,6% Methylmethacrylat (MMA), 2% N,N-dimethyl-p-toluidin und 20 ppm Hydrochinone) und Polyisobutylen (PIB; M_{w} 300 - 3000 g/mol) gemischt. Es werden 20% PMMA, 50% PIB und 30% MMA eingesetzt. Das PIB wirkt als molekularer Weichmacher und das PMMA als Füllstoff, der den Einsatz von geringeren Mengen MMA ermöglicht. Reines MMA würde unter erheblicher Wärmeentwicklung (Trommesdroff-Effekt) polymerisieren. Zu dieser Mischung werden 25 ml Triethanolamin und 5 - 10 ml N-Vinylpyrrolidon gegeben, in denen 0,3% Eosin Y gelöst wurde.

### Beispiel 2 (Selbstaushärtende Polymerlösung)

Eine flüssige Phase besteht aus 97,6% Methylmethacrylat (MMA) als Monomer, 2,4% N,N-dimethyl-p-toluidin als Aktivator und 20 ppm Hydrochinone als Stabilisator. Das Polymerpulver besteht aus 64,4% Polymethylmethacrylat (PMMA), 0,6% Dibenzoylperoxid als Initiator der Polymerisation, 25% Bariumsulfat als Röntgenmarker und 10% Füllstoff. Als Füllstoff kann als mechanisch nicht elastisches Material, wie in Knochenzement, Hydroxylapatit zum Einsatz kommen. In der Folge sind die ausgehärteten Ringstrukturen mechanisch sehr starr und knochenähnlich. Als Füllstoff kann ferner ein mechanisch elastisches Material wie Gummipartikel zum Einsatz kommen. Abhängig vom Anteil sind die mechanischen Eigenschaften der ausgehärteten Ringstruktur gezielt einstellbar.

Als Präpolymere sind neben dem Polyisobutylen auch Elastomere wie der Butylkautschuk (Isobuten-Isopren-Kautschuk; IIK) geeignet, insbesondere das über kationische Polymerisation aus Fällungspolymerisation gewonnene IIK. Weitere Präpolymere oder Latices können einfach über eine Suspensionspolymerisation erzeugt werden. Die Teilchengröße kann dabei zwischen 2 - 200 µm liegen. Bevorzugt sind Teilchen im Bereich von 10 - 50 µm. Eine gute Übersicht über Plastisol oder Organosole ist in WO 2008/019899 gegeben. Hier werden einige Methacrylat-Verbindungen beschrieben, die nach Verarbeitung nachhärten. Nach dem Zusammenbringen der flüssigen Phase mit den anderen Substanzen wird die Polymerisation initiiert und innerhalb ca. 10 Minuten kommt es zu einem Aushärten des Materials.

### Beispiel 3 (Selbstaushärtende Polymerlösung)

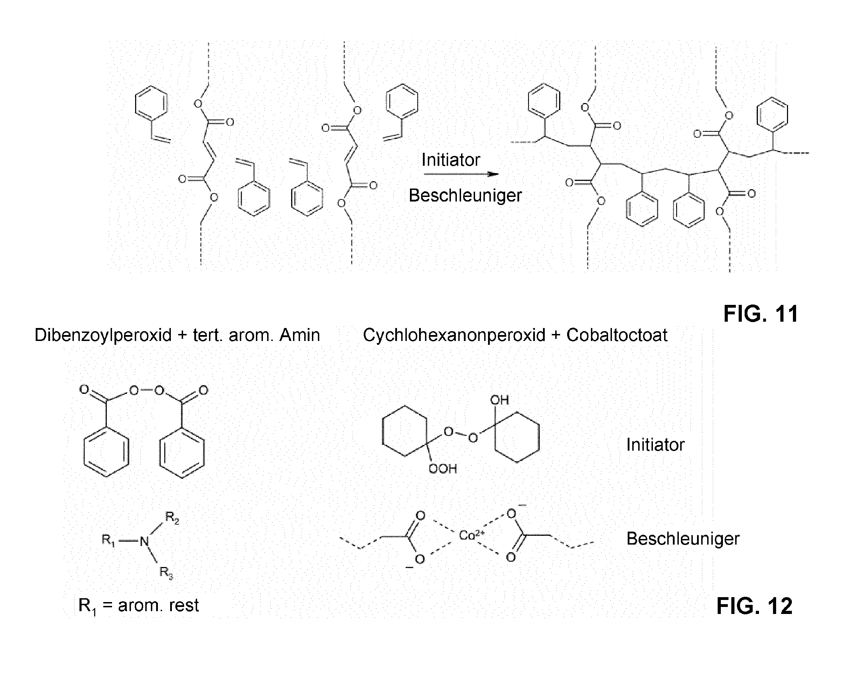

Ein weiteres Beispiel für ein selbstaushärtendes Polymer sind die ungesättigten Polyester (UP). Die Wirkungsweise geht aus Fig.11 hervor. Das UP enthält Monomere (Styrol im vorliegenden Beispiel). Die Vernetzung und die Versteifung geschieht über UV induzierte nachträgliche Vernetzungsreaktion, dabei sind intra- oder intermolekulare Vernetzungen der UP denkbar. Das Styrol bzw. die anderen verwendeten Monomere fungieren zwar auch als Verdünnungsmittel zwecks Einstellung der Verarbeitungsviskosität, sie sind jedoch nicht als Lösemittel im eigentlichen Sinne anzusehen, da sie durch die chemische Härtungsreaktion zum Bestandteil des polymeren Netzwerkes werden. Man bezeichnet sie daher besser als "Reaktivverdünner". Der Verlust durch Verdunsten ist in der Regel sehr gering. Die Herstellung von UP's ist bekannt. Sie werden über Kondensationsreaktionen von ungesättigten Dicarbonsäuren oder ungesättigten Carbonsäureanhydriden mit Diolen gebildet. Die bevorzugte Molmasse liegt bei 6.500 - 13.000 g/mol. Beim Einsatz von 112 ml des UP (10.000 g/mol) werden 16,5 ml Styrol zugegeben. Die Härtung bei Körpertemperatur erfordert normalerweise den Zusatz eines sog. Beschleunigers zum ungesättigten Polyestersystem, da ansonsten entweder keine Härtung erfolgt oder die Aushärtezeit Tage bis Wochen in Anspruch nehmen kann. Zur Beschleunigung von UP-Harzsystemen gibt es hauptsächlich zwei Initiator-Beschleuniger-Kombinationen, nämlich die konventionelle Härtung von UP-Harzen mittels Dibenzoylperoxid/tert. aromatisches Amin und mittels Cyclohexanon/Kobaltoktoat, wie dies in Fig. 12 gezeigt ist.

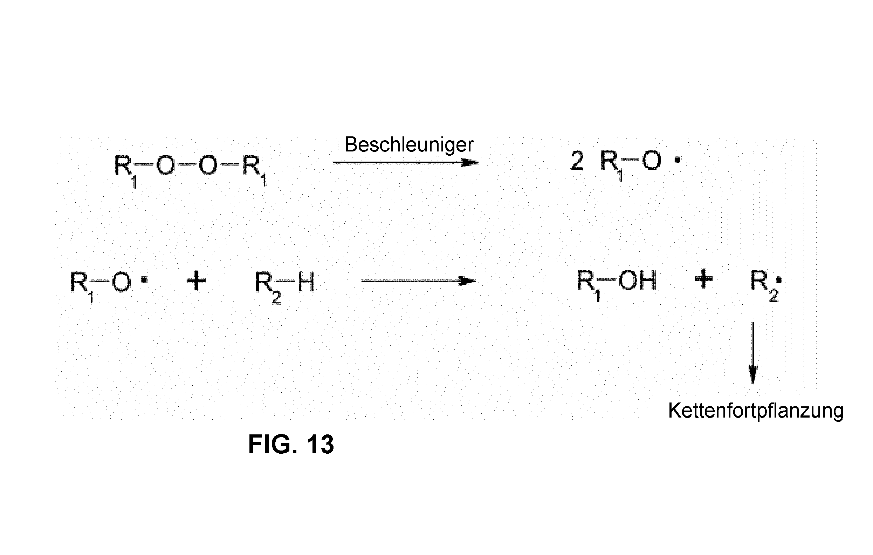

Unter Initiatoren versteht man in der makromolekularen Chemie organische Peroxide, die durch ihren Zerfall in Radikale die Polymerisation von ungesättigten Verbindungen einleiten können. Bei den üblichen Verarbeitungstemperaturen von konventionell gehärteten, ungesättigten Polyesterformulierungen findet die Radikalbildung ohne Beschleuniger nicht oder nur sehr langsam statt. Die sog. Beschleuniger katalysieren den Zerfall der Peroxide durch Herabsetzung der Dissoziationsenergie und leiten gemäß wie in Fig. 13 gezeigt die Reaktion ein.

Die konventionelle Härtung von UP-Harzen mittels Dibenzoylperoxid/tert. aromatisches Amin erfolgt folgendermaßen: Der Zerfall von Diacylperoxiden wie z. B. Dibenzoylperoxid wird durch tertiäre aromatische Amine katalysiert. Dieses System weist eine hohe Reaktivität auf und eignet sich somit zur Härtung von ungesättigten Polyesterformulierungen auch bei relativ niedrigen Temperaturen (bis zu ca. 5°C). Der Peroxidhärter kann nachträglich als letzter Bestandteil in den Polymerschlauch gegeben werden; dadurch wird sichergestellt, dass das UP, unreaktiv vorliegt. Reines Peroxid wäre aufgrund der leichten Zersetzbarkeit bei Temperaturen oberhalb von 25°C bzw. bei Einwirkung von direktem Sonnenlicht zu gefährlich in seiner Handhabung. Daher wird die entsprechende Menge Härter (0,015 mol für den oben angegebenen Ansatz) in einem Lösungsmittel (Ethanol; 2 - 3%) kurz vor der Verarbeitung in die Schlauchstruktur eingebracht, für die Mischung reichen die natürlichen Kontaktreaktionen des Herzmuskels aus. Die Verarbeitungszeit eines solchen Systems ist normalerweise auf ca. 4 - 6 Minuten eingestellt.

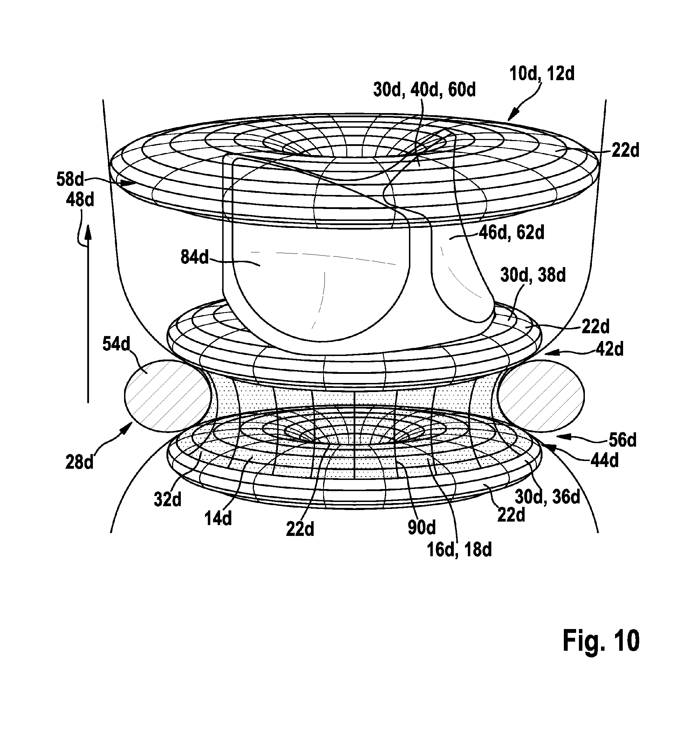

In der Fig. 10 ist ein drittes alternatives Klappenimplantat 10d bzw. ein Herzklappenimplantat 12d zur Implantation im tierischen und/oder menschlichen Körper mit einer künstlichen Klappe 46d bzw. Aortenklappe 62d und mit einem Grundkörper 14d, ausgeführt als ein Stent 32d, gezeigt. Der von einem elastischen Polymerschlauch gebildete Grundkörper 14d, weist einen Bereich 18d auf, der aus einem mittels UV-Strahlung aushärtbaren Material 22d gefertigt ist, wobei der Bereich 18d mit einer Wand 16d und dem kompletten Grundkörper 14d übereinstimmt. Die Klappe 46d ist in einer axialen Richtung 48d über einer Ringstruktur 38d an einem proximalen Ende 42d des Grundkörpers 14d in der Aorta über einer Implantationsstelle 28d bzw. einem Annulus 54d angeordnet. Zu einer oberen Fixierung von Segeln 84d der Klappe 46d ist an dem proximalen Ende 58d der Klappe 46d eine mechanische Stützstruktur 60d in der Form einer dritten Ringstruktur 40d angeordnet. Diese Ringstruktur 40d ist baugleich zu Mitteln 30d zur Verstärkung bzw. Ringstrukturen 36d, 38d an dem proximalen Ende 42d und einem distalen Ende 44d ausgeführt und kann mit demselben aushärtbaren Material 22d wie die Ringstrukturen 36d, 38d oder mit einem anderen Material 22d befüllt werden. Ferner kann zwischen der Ringstruktur 40d und einer oder beiden Ringstrukturen 36d, 38d eine Kommunikationsstruktur analoge einer Kommunikationsstruktur 56d mit hohlen Elementen 90d zwischen den Ringstrukturen 36d, 38d vorgesehen sein (nicht gezeigt).

### Bezugszeichenliste

- 10: Klappenimplantat
- 12: Herzklappenimplantat
- 14: Grundkörper
- 16: Wand
- 18: Bereich
- 20: UV-Strahlung
- 22: Material
- 24: Innenquerschnitt
- 26: Querschnittsfläche
- 28: Implantationsstelle
- 30: Mittel
- 32: Stent
- 34: Stent
- 36: Ringstruktur
- 38: Ringstruktur
- 40: Ringstruktur
- 42: Ende
- 44: Ende
- 46: Klappe
- 48: Richtung
- 50: Fließrichtung
- 52: Fließmedium
- 54: Annulus
- 56: Kommunikationsstruktur
- 58: Ende
- 60: Stützstruktur
- 62: Aortenklappe
- 64: Hohlzylinder
- 66: Mantelfläche
- 68: Umfangsrichtung
- 70: Länge
- 72: Lichtquelle
- 74: Ballonkatheter
- 76: Ballon
- 78: Abgang
- 80: Kalzifizierung
- 82: Aortenwand
- 84: Segel
- 86: Implantationsrichtung
- 88: Umhüllung
- 90: Element
- 92: Metallgerüst

## Patentansprüche

1. Medizinisches Klappenimplantat (10a-d), insbesondere Herzklappenimplantat (12a-d), zur Implantation im tierischen und/oder menschlichen Körper mit einem Grundkörper (14a-d), der zumindest eine Wand (16a-d) aufweist, **dadurch gekennzeichnet, dass** zumindest ein Bereich (18a-d) der zumindest einen Wand (16a-d) des Grundkörpers (14a-d) aushärtbar ist.

2. Medizinisches Klappenimplantat nach Anspruch 1, **dadurch gekennzeichnet, dass** der Bereich (18a-d) der zumindest einen Wand (16a-d) des Grundkörpers (14a-d) mittels UV-Strahlung (20a-d) aushärtbar ist.

3. Medizinisches Klappenimplantat nach Anspruch 1 oder 2, **dadurch gekennzeichnet, dass** der zumindest eine Bereich (18a-d) der zumindest einen Wand (16a-d) des Grundkörpers (14a-d) aus einem aushärtbaren Material (22a-d) ausgewählt ist aus einer Gruppe bestehend aus: Acrylate, Methacrylate, Cyanacrylate, Epoxide, Urethane, Acrylamide, Acrylsäuren hergestellt ist.

4. Medizinisches Klappenimplantat nach einem der vorhergehenden Ansprüche, **dadurch gekennzeichnet, dass** der Grundkörper (14a-d) dazu vorgesehen ist, einen Unterschied in einer Form eines Innenquerschnitts (24a-d) des Grundkörpers (14a-d) und einer Querschnittsfläche (26a-d) einer Implantationsstelle (28a-d) auszugleichen.

5. Medizinisches Klappenimplantat nach einem der vorhergehenden Ansprüche, **dadurch gekennzeichnet, dass** der Grundkörper (14a-d) zumindest eine Mittel (30a-d) zur mechanischen Verstärkung aufweist.

6. Medizinisches Klappenimplantat nach einem der vorhergehenden Ansprüche, **dadurch gekennzeichnet, dass** der Grundkörper (14a-d) einen Stent (32a-d, 34b) umfasst.

7. Medizinisches Klappenimplantat nach Anspruch 5 oder 6, **dadurch gekennzeichnet, dass** das Mittel (30c, 30d) zur mechanischen Verstärkung von zumindest einer Ringstruktur (36c, 36d; 38c, 38d; 40d) gebildet ist.

8. Medizinisches Klappenimplantat nach Anspruch 7, **dadurch gekennzeichnet, dass** die Ringstruktur (36c, 36d; 38c, 38d) an einem proximalen Ende (42c, 42d) und/oder einem distalen Ende (44c, 44d) des Grundkörpers (14c, 14d) angeordnet ist.

9. Medizinisches Klappenimplantat nach Anspruch 7 oder 8, **dadurch gekennzeichnet, dass** die Ringstruktur (36c, 36d; 38c, 38d, 40d) mit einem aushärtbaren Material (22c, 22d) befüllbar ist.

10. Medizinisches Klappenimplantat nach einem der vorhergehenden Ansprüche, **gekennzeichnet durch** zumindest zwei Ringstrukturen (36c, 36d; 38c, 38d, 40d), die unabhängig voneinander befüllbar sind.

11. Medizinisches Klappenimplantat nach einem der Ansprüche 8 bis 10, **gekennzeichnet durch** eine Klappe (46c, 46d), die in einer axialen Richtung (48c, 48d) über der Ringstruktur (38c, 38d) am proximalen Ende (42c, 42d) des Grundkörpers (14c, 14d) angeordnet ist.

12. Medizinisches Klappenimplantat nach einem der vorhergehenden Ansprüche, **dadurch gekennzeichnet, dass** zumindest zwei Ringstrukturen (36c, 36d; 38c, 38d) im bestimmungsgemäßen Endzustand in Fließrichtung (50c, 50d) eines Fließmediums (52c, 52d) axial vor und hinter einem Annulus (54c, 54d) angeordnet sind.

13. Medizinisches Klappenimplantat nach einem der vorhergehenden Ansprüche, **dadurch gekennzeichnet, dass** zwischen zwei Ringstrukturen (36c, 36d; 38c, 38d, 40d) zumindest eine Kommunikationsstruktur (56c, 56d) angeordnet ist.

14. Medizinisches Klappenimplantat nach einem der vorhergehenden Ansprüche, **dadurch gekennzeichnet, dass** zumindest an einer Ringstruktur (38c) an einem proximalen Ende (42c) des Grundkörpers (14c) und/oder an einem proximalen Ende (58d) einer Klappe (46d) eine Stützstruktur (60c, 60d) angeordnet ist.

15. Medizinisches Klappenimplantat nach einem der vorhergehenden Ansprüche, **gekennzeichnet durch** eine Ausgestaltung als Aortenklappe (62a-d).
